# EUROPEAN PATENT SPECIFICATION

(11) **EP 2 629 779 B1**
(45) Date of publication and mention of the grant of the patent: **21.06.2017**
(21) Application number: 11834974.5
(22) Date of filing: 18.10.2011
(51) Int. Cl.: A61K 38/14, A61K 45/06, A61K 9/06, A61K 31/7036, A61K 31/7052, A61K 9/00

(54) **USE OF STORAGE STABLE VISCOUS PHOSPHOLIPID DEPOT TO TREAT WOUNDS**
VERWENDUNG EINES LAGERSTABILEN VISKÖSEN PHOSPHOLIPID-DEPOTS ZUR WUNDBEHANDLUNG
UTILISATION D'UN DÉPÔT VISQUEUX DE PHOSPHOLIPIDES STABLE PENDANT LE STOCKAGE POUR TRAITER LES PLAIES

(30) Priority: 22.10.2010 US 405715 P
(43) Date of publication of application: 28.08.2013
(73) Proprietor: Dr. Reddy's Laboratories SA., 4051 Basel (CH)
(72) Inventor: CHEN, Hailiang, San Diego California 92130 (US); CHEN, Andrew Xian, San Diego California 92130 (US); SURAKANTI, Dushyanth, Bridgewater New Jersey 08807 (US); OKUMU, Franklin, Oakland California 94611 (US)
(74) Representative: Bates, Philip Ian
(86) International application number: PCT/US2011/056661
(87) International publication number: WO 2012/054447

(56) References cited:
- EP-A1- 2 055 309
- US-A- 4 610 868
- US-A- 5 654 337
- US-A1- 2004 147 534
- US-A1- 2004 151 765
- US-A1- 2006 008 480
- US-A1- 2009 053 290
- US-A1- 2009 169 632
- US-A1- 2009 214 628
- US-B1- 6 413 556

## Description

### CROSS REFERENCE TO RELATED APPLICATIONS

This application claims the benefit of U.S. Provisional Application No. 61/405,715, filed October 22, 2010.

### BACKGROUND OF THE INVENTION

A wound is an injury to the body, as from violence, accident, or surgery, that typically involves laceration or breaking of a membrane and usually damages to underlying tissues, and/or bones. Merriam-Webster's Collegiate Dictionary, 11th ed. 2004.

The treatment of a wound depends on the type, cause, location and depth of the wound as well as which other structures beyond the skin are involved. But, generally the treatment involves cleaning, treating with an antibiotic, protecting the wound by covering it with a gauze dressing pad to absorb fluids and prevent further contamination and/or closing the wound to prevent further contamination. Wound closure typically involves using a suture, metal clips, staples and adhesive strips.

Generally, the risk of infection in a wound is reduced by adequate wound cleansing and/or debridement by removing any foreign material and non-viable tissue before application of an antibiotic at the wound.

Vacuum-assisted wound closure systems, which are wound closure systems that continuously or intermittently apply subatmospheric pressure to the surface of a wound, may be used to remove wound fluid and non-viable tissue, to decrease the level of bacteria in the wound, to improve blood flow in the wound bed and surrounding tissue and/or pull the wound edges together and stimulate cell growth. Such vacuum-assisted wound closure systems are described in, for example, U.S. Patents 4,949,880, 5,100,396, 5,261,893, 5,527,293, 6,071,267, 7,799,004, and Gestring, Negative Pressure Wound Therapy, UpToDate, May 2010.

A depot is a way of administering an active ingredient, such as an antibiotic, into the body of a patient for systemic or local action. It is generally administered by subcutaneous or intramuscular injection or instillation into other body tissues, vessels or cavities. A depot can also be applied to a wound before it is staunched, stitched, bandaged or otherwise closed. Unlike removable depots, biodegradable depots disintegrate or degrade within a pre-defined time, typically after the entrapped active pharmaceutical ingredient has been delivered. In other constructs, the biodegradable injectable depot releases its active pharmaceutical ingredient roughly simultaneously with, or as a function of, its gradual degradation. A key advantage of certain biodegradable delivery depots is their ability to deliver medication directly to the intended site of action providing elevated local concentrations of medication when compared to systemic levels.

Depots can also modulate delivery of medication to enable various release profiles. The release profile could be immediate release (burst) followed by a steady state, could be, among others, "zero order" or constant rate of delivery, could provide a slow rise to steady state, or could even provide for a delayed release. In addition, depots have the advantage of allowing release over an extended period of time, with a single administration. Blood levels are not compromised by, for example, patient compliance issues.

Depots can be comprised of particulate systems such as microsphere-based depots and nanosphere-based depots, or can also be comprised of a biodegradable gel, typically made from soluble matrix formers (polymers, lipids, carbohydrates) and either an organic solvent or a mixture of water miscible and non-miscible solvents.

Phospholipids have been used to prepare depots comprising a lipophilic pharmacological active agent. Phospholipids are soluble in oils or organic solvents but insoluble in water. To form a depot, a high concentration of depot-forming phospholipids is often required. This can impact the volume and viscosity of the resulting depot and, accordingly, currently available phospholipid depots can be very difficult to inject through a conventional needle or a syringe. References describing phospholipids-based formulations include WO 89/00077, WO 02/32395, EP 0282405 and U.S. Patent Nos. 5,863,549, 4,252,793, 5,660,854, 5,693,337, and Wang et al., Lyophilization Of Water-In-Oil Emulsions To Prepare Phospholipid-Based Anhydrous Reverse Micelles For Oral Peptide Delivery, 39 European Journal of Pharmaceutical Sciences, at 373-79 (2010).
US4610868 is directed to lipid matrix carriers which provide for the sustained release of bioactive agents in vivo or in vitro.

Vancomycin is a glycopeptide antibiotic used in the prophylaxis and treatment of infections caused by Gram-positive bacteria. It is generally the drug of choice for serious infection and endocarditis caused by S. aureus, coagulase-negative staphylococci, streptococcus pneumoniase, β-hemolytic streptococci, corynebacterium group JK, viridans streptococci, or enterococci when β-lactams cannot be used because of drug allergy or resistance. Vancomycin can be combined with other antimicrobials when treating, *inter alia,* methicillin-resistant coagulase-negative staphylococcal prosthetic valve endocarditis, and enterococcal endocarditis. It has also been used as an alternative agent for pneumococcal meningitis caused by strains with reduced penicillin sensitivity. Vancomycin has been used in cardiac and vascular surgery to prevent post surgical infection. *See* Rybak et al., Vancomycin Therapeutic Guidelines: A Summary of Consensus Recommendations From The Infectious Diseases Society of America, The American Society Of Health-System Pharmacists, and The Society Of Infectious Disease Pharmacists, CID 2009:49 (1 August), pg. 325.

Gentamicin is an aminoglycoside antibiotic used to treat many types of bacterial infections particularly those caused by Gram-negative bacteria. It has been used in a surgical setting because it acts against pathogens such as anaerobes and enterococci. Gentamicin has been used in other surgical applications (e.g. orthopedic settings) and is currently being used in a biodegradable collagen implant.

Both vancomycin and gentamicin in their currently available and commonly used salt forms, e.g. vancomycin hydrochloride and gentamicin sulfate are very hydrophilic antibiotics. These antibiotics, particularly in their salt forms are also difficult to formulate into injectable depots based on phospholipids or other high oil phase content formulations, as they are not freely soluble in phospholipid or oil.

In addition, by conducting a series of stability tests, it has now been found that vancomycin and gentamicin degrade by different mechanisms. Vancomycin loses its stability through hydrolysis and gentamicin degrades due to oxidation or adduct formation. Thus, formulations containing either one of the actives are generally sensitive to these conditions. Moreover, both vancomycin and gentamicin are heat-sensitive and cannot be sterilized by using heat, such as autoclaving or gamma-radiation.

Accordingly, attempting to formulate a depot comprising a vancomycin salt, gentamicin salt or both along with a phospholipid and oil provide many practical challenges. One such attribute includes the formulation should not feature high viscosity since the formulation has to be sterilized by filtering through a sterilizing membrane, such as one having pores of about 0.22 micron or less. There also remains certain dichotomous problems. For instance, these actives also have compatibility problems with phospholipids which, like viscosity, suggests a need to keep phospholipid content low. However, the need for coherent and cohesive gel formation and proper release characteristics suggest just the opposite.
Accordingly, there remains an unmet need for storage stable viscous phospholipid depots containing a vancomycin, gentamicin, a pharmaceutical salt thereof or a mixture thereof that can be administered by subcutaneous or intramuscular injection or by injection or instillation I nto a wound or other body tissues, vessels or cavities to treat and/or prevent wound infection.

### BRIEF SUMMARY OF THE INVENTION

One aspect of the present invention provides a process for making a depot comprising a hydrophilic water-soluble pharmaceutically active agent(s) comprising: (1) dissolving, a pharmaceutically acceptable salt of vancomycin, gentamicin or a mixture thereof in water; (2) forming an oil-in-water emulsion comprising a phospholipid, an oil, and an aqueous solution comprising the pharmaceutically acceptable salt of vancomycin, gentamicin, or the mixture thereof (an "emulsion"); (3) homogenizing the emulsion using a high-pressure homogenizer, such as a MICROFLUIDIZER to obtain a "monophasic solution," (4) ensuring that the pH of the emulsion and/or the monophasic solution is between about 3 to about 6, a range of from about 3 to about 5, or a range of from about 3 to about 4 by adjusting the pH as necessary, (5) lyophilizing the pH adjusted solution, (6) adding a viscosity modifying agent in an amount sufficient to obtain a desired viscosity, (7) pre-filtering of the viscosity modified solution to obtain a clear solution, (8) removing some amount of the viscosity modifying agent from the clear solution to obtain a depot having from about 5.5 wt% to about 7.5 wt % of the viscosity modifying agent relative to the total weight of the depot, (9) sterilizing the depot without heating the depot. In another embodiment, pre-filtering and removing the viscosity modifying agent are optional steps. The pharmaceutically active agent(s) is a pharmaceutically acceptable salt of vancomycin or gentamicin. In some embodiments, the pharmaceutically active agent(s) is a mixture of a pharmaceutically acceptable salt of vancomycin and gentamicin. Depots comprising the pharmaceutically acceptable salts of vancomycin, gentamicin or a mixture thereof are also contemplated, whether made by this process or by some other.

In an embodiment, the method further comprises a step of aseptically filling the viscous depot in a syringe, a vial or any other appropriate device capable of storing and/or delivering the depot to the treatment site or wound.

In accordance with another aspect of the invention, a stabilizing agent is optionally dissolved in water along with the pharmaceutically acceptable ingredient(s). Examples of the stabilizing agent includes, but not limited to EDTA disodium, glycine, L-histidine, citric acid, mithionine, ascorbic acid, L-cysteine, alpha-tocopherol, and mixtures thereof. In yet another aspect of the invention, the depot does not include a stabilizing agent.

In yet another embodiment, the step of homogenizing the emulsion is performed, which results in a primary emulsion, wherein the lipid/oil discontinuous phase has an average diameter of less than about 200 nm, less than about 100 nm, or less than about 80 nm.

The reduction of average diameter of the lipid droplets is believed, without limitation, to reduce the viscosity of the resulting monophasic solution, allowing sterilization through a filter, rather than by using a heat-based sterilization system, such as by autoclaving or gamma-radiation sterilization, which can affect stability of vancomycin and/or gentamicin.

Before the step of homogenization, the emulsion is generally a white, opaque, thick yogurt-like mass. After homogenization, the resulting monophasic solution is generally clear, translucent, and water-like in viscosity and flow properties.
Although the present invention is not limited by any particular theory of operation, it is believed that a very hydrophilic form of vancomycin, gentamicin, or a mixture thereof, can be formulated with phospholipids to form a monophasic solution as defined herein resulting in storage stable viscous depots with desirable properties. It is believed that the extremely small lipid droplets provided during homogenization may be instrumental in the eventual properties of the depots produced, among other factors that may be involved.
In accordance with another embodiment of the present invention, the pH of the monophasic solution is from about 3 to about 6, a range of about 3 to about 5, or a range of from about 3 to about 4 in other embodiments of the invention.
In accordance with the present invention, the pH of the viscous depot, the final product, is from about 3 to about 5, or a range of from about 3 to about 4.
Another aspect of the present invention provides a clear depot comprising a hydrophilic water-soluble pharmaceutically active agent(s) selected from the
group consisting of a pharmaceutically acceptable salt of vancomycin, gentamicin, and a
mixture thereof; water; a phospholipid; an oil; a pH adjusting agent; and a viscosity modifying
agent selected from the group consisting of ethanol, isopropanol, and a mixture thereof; for
use in the prevention and/or treatment of a wound infection; wherein the water present in
the viscous depot is no more than 2 wt % relative to the total weight of the depot; and the
pH of the depot is from 3 to 5.
In another embodiment, the depot is syringeable.
In one embodiment of the present invention, the depot comprises pharmaceutical salts of one or both vancomycin and gentamicin. In another embodiment, the depot comprises pharmaceutically acceptable salts of either vancomycin or gentamicin. In yet another embodiment, the depot comprises a pharmaceutical salt of either vancomycin or gentamicin.

The depots in accordance with the present invention are "clear." This offers advantages in being able to see entrapped air, foreign bodies, and the like to prevent the unintended introduction of same into the body. Interestingly, it has also been discovered that when pharmaceutically acceptable salts of both vancomycin and gentamicin are present in the depot, the depot of the invention is clearer than when the depot contains a pharmaceutically acceptable salts of either vancomycin or gentamicin alone. In such embodiment where pharmaceutically acceptable salts of both vancomycin and gentamicin are present in the depot, the clarity of such depot is "ultra clear" as defined herein. In an embodiment where the depot comprises pharmaceutically acceptable salts of either vancomycin or gentamicin, the clarity of such depot is "translucent" or "clear" as defined herein.

In another embodiment, the viscosity modifying agent is ethanol, wherein the amount of ethanol present in the depot is from about 3 wt % to about 25.0 wt %, about 4 wt % to about 10 wt %, or about 5 wt % to about 6.5 wt % relative to the total weight of the composition.

In yet another embodiment, the amount of phospholipid present in the depot is from about 5 wt % to about 95 wt %, about 25 wt % to about 75 wt %, or about 35 wt % to about 60 wt % relative to the total weight of the composition.

In accordance with another embodiment of the present invention, the amount of oil present in the depot is from about 5 wt % to about 95 wt %, from about 25 wt % to about 75 wt %, or from about 35 wt % to about 60 wt % relative to the total weight of the composition.
In accordance with an embodiment of the present invention, no more than about 80%, no more than about 50%, or no more than about 20% of vancomycin and/or gentamicin is released at two hours when measured in accordance with a USP method I using 500 ml of deionized water as a medium.
In accordance with another aspect of the invention, the depot optionally comprises a stabilizing agent to improve the stability of vancomycin, gentamicin or both. Examples of the stabilizing agent include, but not limited to EDTA (ethylenediaminetetraacetic acid), disodium edetate, glycine, L-histidine, citric acid, methionine, ascorbic acid, L-cysteine, alpha-tocopherol, and mixtures thereof. In accordance with yet another aspect of the invention, the viscous depot does not contain a stabilizing agent. In still another embodiment, the amount of stabilizing agent used, if any, will not negatively impact the stability of each active, vancomycin or gentamicin, in the depot.
In another aspect of the invention, a viscous depot is provided in an applicator, syringe, vial or any other device capable of storing and/or delivering the depot to the treatment site, depot site or wound.
The clear depot for use according to the invention may be administered via intradermal, intramuscular, subcutaneous, instillation or topically.
The clear depot of the present invention may be for use in a method of preventing and/or treating post surgical infection. The clear depot of the present invention may be for use in a method of preventing and/or treating a wound infection by introducing a depot of the present invention to a wound. In an embodiment of the present invention, the wound is selected from the group consisting of a surgical wound, orthopedic wound, traumatic wound, a combat wound and any combination thereof. In accordance with an aspect of the present invention, the surgical wound includes, but not limited, an incised wound, which is clean cut by a sharp instrument.
In another aspect of the present invention, the orthopedic wounds include, but not limited to, injuries to the musculoskeletal system, extremities, pelvis, spine and associated structures, and any combination thereof.
In yet another aspect of the present invention, the traumatic wounds include, but not limited to, open wounds to head, face, chest, abdomen, extremities, pelvis, and/or external and/or injuries with torn and irregular edge with presence of foreign matter and/or non-viable tissue fragments, such as laceration, abrasions, puncture wounds, penetration wounds, and any combination thereof.
In another aspect of the present invention, the combat wounds include, but not limited to, injuries inflicted by an explosive device and/or a weapon, gunshot wounds, any of the traumatic wounds mentioned above, and any combination thereof.

### BRIEF DESCRIPTION OF THE DRAWINGS

Figure 1 is a process flow diagram of an embodiment of the method of making an inventive composition in accordance with an aspect of the invention.
Figure 2 shows the assay recovery of vancomycin and gentamicin of the formulation of EXAMPLE 1 after the autoclave treatment.
Figure 3 is an in vitro release profile of gentamicin and vancomycin of the formulation of EXAMPLE 6 using USP method I.
Figure 4 illustrates plasma concentrations of vancomycin of the formulation of EXAMPLE 1 in rabbits.
Figure 5 illustrates tissue concentrations of vancomycin of the formulation of EXAMPLE 1 in rabbits.
Figure 6 illustrates plasma concentrations of gentamicin of the formulation of EXAMPLE 1 in rabbits.
Figure 7 illustrates tissue concentrations of gentamicin of the formulation of EXAMPLE 1 in rabbits.
Figure 8 illustrates mean vancomycin plasma concentrations in rabbits after single SC wound instillation of the formulation of EXAMPLE 6.
Figure 9 illustrates mean total plasma concentration of gentamicin of the formulation of EXAMPLE 6 in rabbits.

### DETAILED DESCRIPTION

The present invention will be described in more detail below.

While the specification concludes with the claims particularly pointing and distinctly claiming the invention, it is believed that the present invention will be better understood from the following description. All percentages and ratios used herein are by weight of the total composition and all measurements made are at 25°C and normal pressure unless otherwise designated. All temperatures are in Degrees Celsius unless specified otherwise. The present invention can comprise (open ended) or consist essentially of the components of the present invention as well as other ingredients or elements described herein. As used herein, "comprising" means the elements recited, or their equivalent in structure or function, plus any other element or elements which are not recited. The terms "having," "including," and "comprised of" are also to be construed as open ended unless the context suggests otherwise. As used herein, "consisting essentially of" means that the invention may include ingredients in addition to those recited in the claim, but only if the additional ingredients do not materially alter the basic and novel characteristics of the claimed invention. Generally, such additives may not be present at all or only in trace amounts. However, it may be possible to include up to about 10% by weight of materials that could materially alter the basic and novel characteristics of the invention as long as the utility of the compounds (as opposed to the degree of utility) is maintained. All ranges recited herein include the endpoints, including those that recite a range "between" two values. Terms such as "about," "generally," "substantially," and the like are to be construed as modifying a term or value such that it is not an absolute. Such terms will be defined by the circumstances and the terms that they modify as those terms are understood by those of skill in the art. This includes, at very least, the degree of expected experimental error, technique error and instrument error for a given technique used to measure a value.

Note that while the specification and claims may refer to a final product such as, for example, a depot or other dosage form of the invention as, for example, containing a pH at an intermediate state it may be difficult to tell from the final dosage form that the recitation is satisfied. However, such a recitation may be satisfied if the materials used prior to final production meet that recitation. Similarly, the amount of ingredients introduced into, for example, the emulsion, if described as being by weight may change relative to the weight of the product at some other phase of production such as, in the final depot, which may weight more or less. It is sufficient that those weight percentages were correct at any steps of production and/or in any intermediate. Indeed, as to any property or characteristic of a final product which cannot be ascertained from the dosage form directly, it is sufficient if that property resides in the components recited just prior to final production steps.

The term "emulsion" used herein is a system of two immiscible liquid phases. One of the two phases (the internal phase, discontinuous phase or discrete phase) is distributed as droplets/globules through the second phase (the external or continuous phase). As used herein, emulsions include oil-in-water (O/W) emulsions, in which a less polar liquid commonly referred to as an oil is in the internal phase; and water-in-oil (W/O) emulsions, in which an aqueous or other relatively polar liquid is in the internal phase.

The term "monophasic solution" and "nanoemulsion" are used interchangeably herein. It is noted that the term "solution" in "monophasic solution" does not mean that it is a homogeneous mixture of two or more substances, but that it is a resulting product of the homogenization step using a high-pressure homogenizer, such as a MICROFLUIDIZER.

The term "monophasic," "one phase" and "one phase-like" are used to mean that the resulting product will remain as one phase without separation of phases or precipitation even after 6000 g centrifugation for 10 minutes at 25 deg C in 1 g sample quantity, using a centrifuge made by Heraeus, Model Biofuge Fresco or any equivalent.

The term "viscous" as used herein means that the viscosity of the composition is from about 1 mPa·s (1 centipoise) to about 5000 mPa·s (5000 centipoise), from about 10 mPa·s (10 centipoise) to about 2000 mPa·s (2000 centipoise), or from 100 mPa·s (100 centipoise) to 1500 mPa·s (1500 centipoise).

The term "syringeable" as used herein means that the composition may be administered with a syringe or a catheter or withdrawn from a vial into a syringe. It does not mean, however, that the composition of the invention must actually be in a syringe or administered using a syringe unless the specific recitation or the context suggests that meaning.

The term "translucent" and "clear" are used interchangeably herein to mean that the composition of the invention is not hazy or opaque, and that it is free from visually suspended particles. It should also be free of bubbles. Moreover, by translucent, it is meant that the depot is free from visually suspended particles and should also be free of bubbles. Moreover, by "translucent" or "clear," it is also meant that the depot of the present invention has a light transmittance of greater than about 90% measured at 800 nm (T800) in a 1 cm path quartz cuvette and alcohol as blank when measured by a UV-visible spectrophotometer, such as the one made by Pharmacia, Model Ultrospec III.
By "hazy" or "opaque," it is meant that a T800 value of the depot is less than about 90%.
By "ultra clear," it is meant that a T800 value of the depot is greater than about 92%, or 95%.
The term "stable" as used herein means that (1) the formulation remains clear at 25 deg C for at least one year, or (2) the formulation remains clear and does not separate out or precipitate after centrifugation when the formulation is exposed to 40 deg C for one week.
The term "gel" and "depot" are used interchangeably herein.
One aspect of the present invention provides a clear depot comprising a hydrophilic water-soluble pharmaceutically active agent(s) selected from the group consisting of a pharmaceutically acceptable salt of vancomycin, gentamicin and a mixture thereof, water, a phospholipid, an oil, a pH adjusting agent, and a viscosity modifying agent **selected from the group consisting of ethanol, isopropanol, and a mixture thereof, for use in the prevention and/or treatment of a wound infection,** wherein the water present in the viscous depot is no more than about 2 wt %, e.g. no more than about 0.5 wt % of water relative to the total weight of the depot; and the pH of the depot is from 3 to 5.
The clear depot optionally comprises a stabilizing agent. In another aspect of the invention, the clear depot is provided in a syringe, vial or any other device capable of delivering the depot to the treatment site, depot site or wound.

### Pharmaceutical Active Ingredient

The pharmaceutical active ingredient in accordance with the present invention is a pharmaceutically acceptable salt of vancomycin, gentamicin or a mixture thereof. In one embodiment, the pharmaceutical active ingredient in accordance with the present invention is vancomycin hydrochloride, gentamicin sulfate or a mixture thereof. In another embodiment, the pharmaceutical active ingredients in accordance with the present invention are vancomycin hydrochloride and gentamicin sulfate. In yet another embodiment, the pharmaceutical active ingredient in accordance with the present invention is either vancomycin hydrochloride or gentamicin sulfate.

Examples of the pharmaceutically acceptable salt include, but not limited to, any acids that can form salts with either vancomycin or gentamicin such as acetic acid, hydrochloric acid, hydrobromic acid, citric acid, formic acid, lactic acid, succinic acid, sulfuric acid.

The amount of the pharmaceutical active ingredients that may be present in the viscous depot can vary with a number of parameters including the size of the total intended dose, the duration of administration, the size of the depot and where and how it will be administered, the type of active to be administered, the pattern of administration (*e.g*., continuous, delayed, etc.) and the like. However, generally, the total amount of the pharmaceutically acceptable ingredient may be from about 0.001 wt % to about 50 wt %, from about 0.01 wt % to about 10 wt %, or from about 0.1 wt % to about 5 wt % relative to the total weight of the viscous depot.

### Oil

An oil in accordance with the present invention may be, for instance, natural oils such as vegetable oils, animal oil, vitamin E, vitamin E ester, and the like and/or synthetic or semisynthetic oils, or mixtures thereof.

A vegetable oil refers to oil derived from plant seeds or nuts. Examples of vegetable oils include, but are not limited to, almond oil, borage oil, black currant seed oil, castor oil, safflower oil, soybean oil, sesame oil, cottonseed oil, grapeseed oil, sunflower oil, canola oil, coconut oil, palm oil, orange oil, corn oil, olive oil and the like.

An animal oil refers to triglyceride oil derived from an animal source. Examples of animal oil can be fish oil, or from other sources such as tallow, lard and the like.

Examples of synthetic or semisynthetic oils are mono-, di- or triglycerides, whose acid components are C6 to C20 saturated and/or unsaturated fatty acids, CAPTEX® (various grades of propylene glycol esters such as propylene glycol didecanoate, and glycerol esters such as glyceryl tricaprylate/caprate); MIGLYOL® (caprylic/ capric acid triglycerides; or caprylic/capric/linoleic acid triglycerides; or caprylic/capric/succinic acid triglycerides; or propylene glycol diester of caprylic/capric acid and admixtures with other agents; CAPMUL® (available in different grades, e.g. Capmul MCM. It is mainly mono-and di-esters of glycerol and of propylene glycol, such as glyceryl mono-oleate and propylene glycol monocaprylate. Another grade consists of polyethylene glycol glyceryl monostearate. In one embodiment, the oil used in accordance with the present invention is sesame oil.

The amount of the oil that may be present in the viscous depot may be from about 5 wt % to about 95 wt %, from about 25 wt % to about 75 wt %, or from about 35 % to about 60 % relative to the total weight of the viscous depot.

In certain embodiments, the oil to phospholipid ratio in the viscous depot may be within a range of from about 20: 1 to about 1 : 20, from about 3 : 1 to about 1 : 3, or from about 1 : 2 to about 1 : 1, by weight.

### Phospholipid

Phospholipid in accordance with the present invention refers to a lipid molecule containing one or more phosphate groups, including those derived from either glycerol (phosphoglycerides, glycerophospholipids) or spingosine (sphingolipids).

In some embodiments, phospholipids are triglyceride derivatives in which one fatty acid has been replaced by a phosphate group and one of several nitrogen-containing molecules. The fatty acid chains are hydrophobic and the charges on the phosphate and amino groups make that portion of the molecule hydrophilic. The result is an amphiphilic molecule.

According to the United States Pharmacopoeia (USP), lecithin is a non-proprietary name describing a complex mixture of acetone-insoluble phospholipids, which comprise mainly of phosphatidylcholine, phosphatidylethanolamine, phosphatidylserine and phosphatidylinositol, combined with various amounts of other substances such as triglycerides, fatty acids and carbohydrates. The composition of lecithin and hence its physical properties vary depending upon the source of the lecithin and phospholipid composition, e.g., phosphotidylcholine content, etc.

In accordance with an embodiment of the present invention, lecithin used herein are pharmaceutical grade lecithins derived from egg or soybean, which have been used in parenteral products and are substantially free from irritating, allergenic, inflammatory agents or agents that cause other adverse biological reactions.

In accordance with the practice of the present invention, the selection of phospholipid for preparing the viscous depot is determined based on the ability of the phospholipid to (1) be chemically compatible with the hydrophilic water-soluble pharmaceutically active agent(s) selected from the group consisting of pharmaceutically acceptable salts of vancomycin, gentamicin and a mixture thereof, (2) form a monophasic solution and maintain the small droplet size through the manufacturing process and during storage, and (3) provide the desired depot and provide the desired release of the pharmaceutically active agent.

Examples of the phospholipid include, but not limited to, sphingolipids in the form of sphingosine and derivatives (obtained from soybean, egg, brain and milk), gangliosides, and phytosphingosine and derivatives (obtained from yeast).

Phospholipids can also be synthesized and examples of common synthetic phospholipids include, but not limited to, diglycerols, such as 1,2-diauroyl-sn-glycerol (DLG), 1,2-dimyristoyl-sn-glycerol (DMG), 1,2-dipalmitoyl-sn-glycerol (DPG), 1,2-distearoyl-sn-glycerol (DSG); phosphatidic acids, such as 1,2-dimyristoyl-sn-glycero-3-phosphatidic acid, sodium salt (DMPA, Na), 1,2-dipalmitoyl-sn-glycero-3-phosphatidic acid, sodium salt (DPPA, Na), 1,2-distearoyl-sn-glycero-3-phosphatidic acid, sodium salt (DSPA, Na); phosphocholines, such as 1,2-didecanoyl-sn-glycero-3-phosphocholine (DDPC), 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1,2-dioleoyl-sn-glycero-3-phosphocholine (DOPC), 1,2-dilinoleoyl--sn-glycero-3-phosphocholine (DLOPC), 1,2-dierucoyl-sn-glycero-3-phosphocholine (DEPC), 1,2-dieicosapentaenoyl-sn-glycero-3-phosphocholine (EPA-PC), 1,2-didocosahexaenyl-sn-glycero-3-phosphocholine (DHA-PC), 1-myristoyl-2-palmitoyl-sn-glycero-3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-stearoyl-sn-glycero-3-phosphocholine (PSPC), 1-stearoyl-2-myristoyl-sn-glycero-3-phosphocholine (SMPC), 1-stearoyl-2-palmitoy-sn-glycero-3-phosphocholine (SPPC), 1-myristoyl-2-oleoyl-sn-glycero-3-phosphocholine (MOPC), 1-palmitoyl-2-oleoy-sn-glycero-3-phosphocholine (POPC), 1-stearoyl-2-oleoyl-sn-glycero-3-phosphocholine (POPC); phosphoethanolamines, such as hydrogenated soybean phosphoethanolamine (HSPE), non-hydrogenated egg phosphoethanolamine (EPE), 1,2-dilauroyl-sn-glycero-3-phosphoethanolamine (DLPE); 1,2-dimyristoyl-sn-glycero-3-phosphoethanolamine (DMPE); 1,2-dipalmitoyl-sn-glycero-3-phosphoethanolamine (DPPE); 1,2-distearoyl-sn-glycero-3-phosphoethanolamine (DSPE); 1,2-dioleoyl-sn-glycero-3-phosphoethanolamine (DOPE); 1,2-dilinoleoyl-sn-glycero-3-phosphoethanolamine (DLoPE); 1,2-dierucyl-sn-glycero-3-phosphoethanolamine (DEPE), 1,2-palmitoyl-sn-glycero-3-phosphoethanolamine (POPE); phosphoglycerols such as hydrogenated soy bean phosphatidylglycerol, sodium salt (HSPG, Na), non-hydrogenated egg phosphatidylglycerol, sodium salt (EPG, Na), 1,2-dilauroyl-sn-glycero-3-phosphoglycerol, sodium salt (DLPG, Na), 1,2-dimyristoyl-sn-glycero-3-phosphoglycerol, sodium salt (DMPG, Na), 1,2-dimyristoyl-sn-glycero-3-phospho-sn-1-glycerol, ammonium salt (DMP-sn-1-G, NH₄), 1,2-dipalmitoyl-sn-glycero-3-phosphoglycerol, sodium salt (DPPG, Na), 1,2-distearoyl-sn-glycero-3-phosphoglycerol, sodium salt (DSPG, Na), 1,2-distearoyl-sn-glycero-3-phospho-sn-1-glycerol, sodium salt (DSP-sn-1G, Na), 1,2-dioleoyl-sn-glycero-3-phosphoglycerol, sodium salt (DOPG, Na), 1,2-dierucyl-sn-glycero-3-phosphoglycerol, sodium salt (DEPG, Na), 1,2-palmitoyl-sn-glycero-3-phosphoglycerol, sodium salt (POPG, Na); phosphatidylserines such as 1,2-dimyristoyl-sn-glycero-3-phospho-L-sine, sodium salt (DMPS, Na), 1,2-dipalmitoyl-sn-glycero-3-phospho-L-sine, sodium salt (DPPS, Na), 1,2-distearyl-sn-glycero-3-phospho-L-sine, sodium salt (DSPS, Na), 1,2-dioleoyl-sn-glycero-3-phospho-L-sine, sodium salt (DOPS, Na), 1-palmitoyl-2-oleoyl-sn-glycero-3-phospho-L-sine, sodium salt (POPS, Na); mixed chain phospholipids, such as 1-palmitoyl-2-oleoyl-sn-glycero-3-phosphocholine (POPC), 1-palmitoyl-2-oleoyl-sn-glycero-3-phospoglycerol, sodium salt (POPG, Na), 1-palmitoyl-2-oleoyl-sn-glycero-3-phosphoglycerol, ammonium salt (POPG, NH₄); lysophospholipids, such as 1-myristoyl-2-lyso-sn-glycero-3-phosphocholine (S-lyso-PC), 1-palmitoyl-2-lyso-sn-glycero-3-phosphocholine (P-lyso-PC), 1-stearoyl-2-lyso-sn-glycero-3-phosphocholine (S-lyso-PC); and pegylated phospholipids, such as N-(carbonyl-methoxypolyethyleneglycol 2000)-MPEG-2000-DPPE, 1,2-dipalmitoyl-sn-glycero-3-phosphoethanolamine, sodium salt, N-(carbonyl-methoxypolyethyleneglycol 5000)-MPEG-5000-DSPE, 1-2-distearoyl-sn-glycero-3-phosphoethanolamine, sodium salt, N-(Carbonyl-methoxypolyethyleneglycol 5000)-MPEG-5000-DPPE, 1,2-dipalmitoyl-sn-glycero-3-phosphoethanolamine, sodium salt, N-(carbonyl-methoxypolyethyleneglycol 750)-MPEG-750-DSPE, 1,2-distearoyl-sn-glycero-3-phosphoethanolamine, sodium salt, N-(carbonyl-methoxypolyethyleneglycol 2000)-MPEG-2000-DSPE, 1,2-distearoyl-sn-glycero-3-phosphoethanolamine, sodium salt.

The amount of the phospholipids that may be present in the viscous depot can vary with a number of parameters including the viscosity of final formulation, the duration of administration, the size of the depot and where and how it will be administered, the type of active to be administered, the pattern of administration (*e.g.,* continuous, delayed, etc.) and the like. However, generally, the amount of the phospholipid that may be present in the viscous depot may be from about 5% to about 95% relative to the total weight of the composition, or about 35 % to about 60 % relative to the total weight of the composition.

### Water

Water which can be used in accordance with the present invention includes, but not limited to distilled and deionized water, or any other liquid which is capable of dissolving the hydrophilic water-soluble vancomycin and/or gentamicin and capable of subliming/evaporating during the lyophilization step.

In order to produce a monophasic solution using a high pressure homogenizer, such as a MICROFLUIDIZER, the oil-in-water emulsion must contain from about 10 % to about 80 % water, from about 30 % to about 80 % water, or from about 60 % to 80 % water relative to the total weight of the oil-in-water emulsion in order to have the desired flow property to be processed in the homogenizer, such as a MICROFLUIDIZER.

However, once the monophasic solution is obtained, most of water may be removed by for example, lyophilization, sublimation and/or evaporation.

Vancomycin degrades due to hydrolysis, and the amount of residual water in the final viscous depot affects the long term stability of vancomycin. When vancomycin precipitates, the viscous depot turns from translucent to hazy or separates into two phases as shown in EXAMPLE 2 herein.

Accordingly, in accordance with the present invention, the amount of residual water must be maintained lower than 2 wt % or lower than about 0.5 wt % of water relative to the total weight of the viscous clear depot in order to maintain the vancomycin stable during storage.

### pH Adjusting Agent

The pH adjusting agent in accordance with the present invention is any non-toxic acid, base or salt. Examples of pH adjusting agents include, but not limited to, hydrochloric acid, acetic acid, sulfuric acid, sodium hydroxide, potassium hydroxide, ammonium hydroxide, lysine, arginine, and the like.

As mentioned above, gentamicin degrades due to oxidation or adduct formation. As shown in EXAMPLE 4 hereinbelow, pH affects the long term stability of gentamicin, and when gentamicin precipitates, the viscous depot turns from translucent to hazy.

Accordingly, pH of the viscous depot may be from about 3 to about 5, or a range of from about 3 to about 4.

### Stabilizing Agent

A stabilizing agent in accordance with the present invention is a material which reduces catalytic effect of metal ion on the oxidation, hydrolysis or other degradation reactions and or increases stability of the hydrophilic water-soluble pharmaceutically active agent. Examples of such stabilizing agent include, but not limited to, EDTA (ethylene-diaminetetraacetic acid), disodium edetate, glycine, L-histidine, citric acid, methionine, ascorbic acid, L-cysteine, alpha-tocopherol, and mixtures thereof. In certain embodiments, the amount of the stabilizing agent present in the viscous depot is from about 0.001 % to about 5.0 % relative to the total weight of the composition, or about 0.01 % to about 1.0 % relative to the total weight of the composition. In another embodiment, the viscous depot does not contain a stabilizing agent.

### Viscosity Modifying Agent

A viscosity modifying agent in accordance with the present invention is an aqueous or non-aqueous (other than having a contaminant level of water) liquid which is capable of dissolving the dry paste formed after lyophilization, sublimation and/or evaporation.

Examples of a viscosity modifying agent include, without limitation, ethanol, isopropanol, and a mixture thereof. In one embodiment, the viscosity modifying agent is substantially non-aqueous. In another embodiment, the viscosity modifying agent is ethanol.

The viscosity modifying agent is added to the dry paste until the dry paste is completely dissolved in the agent. The resulting viscosity modified solution may also become "hazy." In one embodiment, the viscosity modifying agent and the dry paste are mixed at a temperature of about 10 deg to 80 deg C, or in a range of about 50 deg C to about 70 deg C, or in a range of about 25 deg C to 60 deg C.

The viscosity modifying agent is added to the dry paste until the amount of viscosity modifying agent is about 10 wt %, 20 wt %, 25 wt % or 30 wt % relative to total weight of the resulting solution. The resulting viscosity of the solution can be from about 10 to about 200 mPa·s (centipoise), from 15 to 100 mPa·s (centipoise), or about 20 mPa·s (centipoise) to about 50 mPa·s (centipoise).

Viscosity can be determined using a Brookfield digital programmable rheometer with the SP-40 spindle or any other equivalent rheometer. More specifically, the starting RPM of the rheometer can be from 0.1 to 1.0, then reducing the RPM to 0.1 in 0.1 RMP increment every 30 seconds. The viscosity measurement can be recorded at 0.8 RMP at an ambient temperature of about 30 deg C.

Subsequently, some amount of the viscosity modifying agent used to dissolve the dry paste may be removed. The removal of the viscosity modifying agent may be done until the residual amount of viscosity modifying agent which may be present in the viscous depot is from about 1% to about 20%, from about 2% to about 18%, or from about 5.0% to about 6.5% relative to the total weight of the viscous depot. If over-dried, the viscosity modifying agent may be added back as needed. The viscosity of the resulting viscous depot in accordance with the present invention is from about 1 mPaS (centipoise) to about 5000 mPaS (centipoise), from about 10 mPaS (centipoise) to about 2000 mPaS (centipoise), or from 100 mPaS (centipoise) to 1500 mPaS (centipoise).

### Process Description

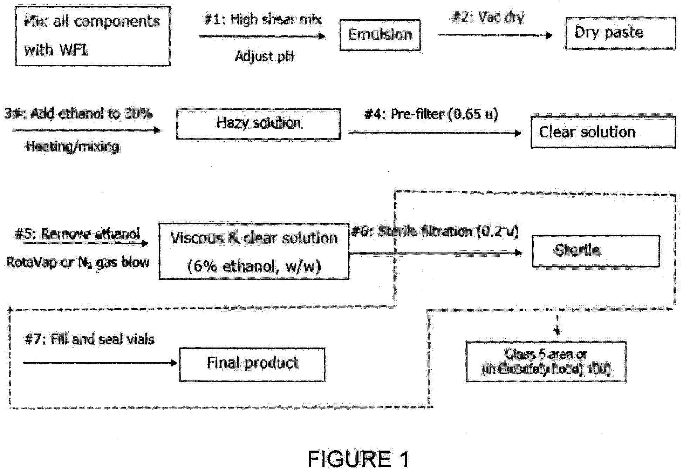

As shown in Figure 1, one aspect of the present invention provides a process for making a viscous depot comprising a hydrophilic water-soluble pharmaceutically active agent selected from the group consisting of vancomycin, gentamicin, and a mixture thereof, comprising: (1) dissolving a pharmaceutically acceptable salt of vancomycin or gentamicin, or a mixture thereof in water to form an aqueous solution; (2) forming an emulsion comprising a phospholipid, an oil, and an aqueous solution comprising the pharmaceutically acceptable salt of vancomycin, gentamicin, or a mixture thereof; (3) homogenizing emulsion using a high-pressure homogenizer, such as a MICROFLUIDIZER to obtain a monophasic solution, (4) ensuring that the pH of the emulsion and/or the monophasic solution is between about 3 to about 6, a range of about 3 to about 5, or a range of from about 3 to about 4, (5) lyophilizing the monophasic solution to form a dry paste, (6) adding a viscosity modifying agent in an amount sufficient to obtain a desired viscosity, (7) pre-filtering of the viscosity modified solution to obtain a clear solution, (8) removing some amount of the viscosity modifying agent from the clear solution to obtain a viscous depot having from about 1 wt % to about 20 wt %, about 2 wt% to about 18 wt %, or about 5.5 wt% to about 7.5 wt % of the viscosity modifying agent relative to the total weight of the viscous depot, and (9) sterilizing the viscous depot without heating. In another embodiment, pre-filtering and removing the viscosity modifying agent are optional steps.

### Dissolving Salts of Vancomycin And/Or Gentamicin In Water

First, vancomycin hydrochloride, gentamicin sulfate or both are dissolved in water to form an aqueous solution.

The initial drug concentration of vancomycin hydrochloride in water is from about 1 mg/ml to about 50 mg/ml or from about 20 mg/ml to about 30 mg/ml, and initial drug concentration of gentamicin sulfate in water is from about 1 mg/ml to about 75 mg/ml, or from about 10 mg/ml to about 30 mg/ml.

### Forming Oil-In-Water Emulsion

Then the aqueous solution of pharmaceutically acceptable salts of vancomycin and/or gentamicin, phospholipid, oil, optionally a pH adjusting agent and optionally a stabilizing agent is mixed to form an oil-in-water emulsion.

### Homogenizing TO Obtain A Monophasic Solution

Subsequently, the emulsion may be homogenized using a high shear mixer and/or a high-pressure homogenizer, such as a MICROFLUIDIZER, to reduce the primary emulsion lipid size to an average diameter of less than 200 nm, less than 100 nm, and or less than 80 nm to form a monophasic solution.

In order to produce such monophasic solution, the oil-in-water emulsion advantageously contains about 10 % to about 80 % water, from about 30 % to about 80% water, or from about 60 % to 80% water relative to the total weight of the oil-in-water emulsion in order to have the desired flow property to be processed in the homogenizer, such as a MICROFLUIDIZER.

### Adjusting pH

The pH may be adjusted by adding a pH adjusting agent before and/or after the step of homogenization so that the pH of the composition is from about 3 to about 6, a range of about 3 to about 5, or a range of from about 3 to about 4.

In another embodiment, this step is performed by adding an appropriate amount of a pH adjusting agent to the emulsion, followed by high shear mixing for about 1 minute. Then, after the homogenization step, the pH of the composition is checked and may be adjusted again if necessary.

### Lyophilization, Sublimation or Evaporation

By removing the water, pharmaceutically acceptable salts of gentamicin and/or vancomycin become uniformly dispersed in the phospholipid/oil vehicle. Water is then removed from the monophasic solution by lyophilization, sublimation and/or evaporation so that the amount of residual water in the resulting dry paste or the final syringeable clear depot is lower than about 2 wt %, or lower than about 0.5 wt % of water relative to the total weight of the dry paste or viscous clear depot. In another embodiment, the emulsion is freeze-dried using a tray lyophilizer.

### Addition of Viscosity Modifying Agent

The viscosity modifying agent is added to the dry paste until the dry paste is completely dissolved and a hazy solution is formed. The viscosity modifying agent may be added to the dry paste until the amount of viscosity modifying agent is about 75 wt %, about 50 wt %, about 30 wt % or about 25 wt % relative to total weight of the hazy solution. In one embodiment, the viscosity modifying agent and the dry paste may be mixed at a temperature of about 10 deg to 80 deg C, or about 25 deg C to 60 deg C.

### Pre-filtration

The hazy solution is then filtered using for example 0.65 micron filter to form a clear solution. The hazy component removed by the pre-filtration steps consists of a small fraction of vancomycin (about 2% target assay) and gentamicin (3-4% target assay). This loss may be compensated by adjusting up the initial load or dropping the assay targets. This is an optional step and is not required for certain embodiments of the invention.

### Removal of Viscosity Modifying Agent

Subsequently, the viscosity modifying agent which was added to dissolve the dry paste is removed. Removal of the viscosity modifying agent may be done until the amount of residual viscosity modifying agent which may be present in the viscous depot from about 1% to about 50%, from about 2% to about 18%, or from about 5% to about 6.5% relative to the total weight of the viscous depot.

If over-dried, the viscosity modifying agent may be added back as needed. Removal of the viscosity modifying agent may be done using a rotary evaporator or by blowing with nitrogen gas or air. Thermal gravimetric Analysis (TGA) can be used to measure the amount of viscosity modifying agent removed from the clear solution to form a viscous depot.

The viscosity of the resulting viscous depot in accordance with the present invention is from about 1 mPa·s (centipoise) to about 5000 mPa·s (centipoise), or from about 10 mPa·s (centipoise) to about 2000 mPa·s (centipoise), or from about 100 mPa·s (centipoise) to about 1500 mPa·s (centipoise). Viscosity measurement can be performed using any conventional method, including using a Brookfield Digital Programmable Rheometer with Model No. DV-III with Spindle No. SP-40. This is an optional step and is not required for certain embodiments of the invention.

### Sterile Filtration

The viscous depot is then sterilized by filtering through a sterilizing membrane, such as one having pores of about 0.22 micron or less.

Another aspect of the present invention is a method of administering via intradermal, intramuscular, subcutaneous, instillation or topically the viscous depot comprising a pharmaceutically acceptable salt of vancomycin, gentamicin, or a mixture thereof, water, phospholipid, an oil, a pH adjusting agent and a viscosity modifying agent.

### Treatment And/Or Prevention Of Wound Infection

An aspect of the present invention is a method of preventing and/or treating a wound infection by administering a depot of the present invention to the wound. In another embodiment, there is provided a method of rendering localized tissue unable to sustain pathogenic microorganism by administering a depot of the present invention to the wound.

Wounds include, but are not limited to, chronic wounds, acute wounds, surgical wounds, orthopedic wounds, traumatic wounds, combat wounds and any combination thereof.

The orthopedic wounds include, but are not limited to, injuries to the musculoskeletal system, extremities including pelvis, spine and associated structures, and any combination thereof.

The traumatic wounds include, but are not limited to, open wounds to head, face, chest, abdomen, extremities (including pelvis), and/or external and/or injuries with torn and irregular edge with presence of foreign matter and/or non-viable tissue fragments, such as laceration, abrasions, puncture wounds, penetration wounds, and any combination thereof.

The combat wounds include, but are not limited to, injuries inflicted by an explosive device and/or a weapon, gunshot wounds, any of the traumatic wounds mentioned above, and any combination thereof.

In another aspect of the present invention, the methods further comprise the steps of cleaning the wound to substantially remove a foreign material and/or non-viable tissue; and closing the wound.

In an embodiment of the present invention, the step of closing the wound is performed using a suture, metal clips, staples or adhesive strips.

In another aspect of the present invention, the methods comprise administering a depot of the present invention to a wound and using a vacuum-assisted wound closure system to the wound.

In yet another aspect of the present invention, the methods comprise cleaning the wound to substantially remove a foreign material and/or non-viable tissue; using a vacuum-assisted wound closure system to the wound; introducing a depot of the present invention to a wound; and closing the wound.

Also contemplated by the present invention is a method of wound closing using a depot of the present invention. The method comprises the step of administering the depot of the present invention either before, during or after closing of the wound using, for example, a suture, metal clips, staples, dressing, bandages and/or adhesive strips/tapes.

### EXAMPLES

### EXAMPLE 1: Formulation In Accordance With The Present Invention And Process Of Making The Formulation

**Table 1:**

| List Of Ingredients Of The Formulation | |
|---|---|
| In Accordance With The Present Invention | |
| Component | w/w% |
| Gentamicin sulfate | Equivalent to 0.36% in the "USP Gentamicin Assay" value |
| Vancomycin hydrochloride | Equivalent to 0.24% in the "USP Vancomycin Assay" value |
| Soy lecithin (PL90G) | 53.3 |
| L-Histidine | 0.1 |
| Ethanol | 6.0 |
| Sesame oil | 40.0 |
| TOTAL | 100% |

One hundred (100) grams of a formulation in accordance with the present invention was prepared as follows: First, a 500 mL beaker was charged with 0.36 g gentamicin sulfate, 0.24 g vancomycin hydrochloride, 53.3 g PL90G, 40 g sesame oil and 0.1 g L-histidine. To this was then added water (mL) for Injection (WFI) and the mixture was homogenization by a high shear mixer at 5000 RPM for 15 min. The resulting monophasic solution was lyophilized to remove water to less than 0.2% residual moisture to obtain a dry paste. This dry paste was mixed with water and/or ethanol, to form a formulation and used in several of the studies, including the EXAMPLE 2 to EXAMPLE 5 as set forth hereinbelow.

### EXAMPLE 2: Effect Of Water Content On Appearance Of Formulation Of EXAMPLE 1

Various amounts of water (from 1.1 wt% to 4.1 wt %) and ethanol (at 6 wt %) were added into the dry paste of EXAMPLE 1 to produce several samples of formulations of EXAMPLE 1. Samples were mixed well by a BeadBeater mixer, centrifuged to remove air bubble, and then observed for initial appearance ("Initial sample"). Also, samples were passed thru 0.45 um filter and the filtrates were stored at 2-8°C for further appearance observation ("Filtered sample"). Table 2 shows the effect of water content on the appearance of the formulations. It was found that water content significantly affected the appearance of the formulations:

**Table 2:**

| Effect Of Water Content On The Appearance Of Example 1 Formulations | | | | | | | | |
|---|---|---|---|---|---|---|---|---|
| Sample ID | S-1 | S-2 | S-3 | S-4 | S-5 | S-6 | S-7 | S-8 |
| Water (%) | 1.14 | 1.46 | 1.83 | 2.05 | 2.61 | 3.06 | 3.70 | 4.07 |
| Initial sample | Hazy | | | | Clear | | | 2 phases |
| Filtered sample | All clear after filtration. However, with more water, a delayed precipitation occurred at 2-8 °C after about 3 to 7 days. | | | | | | | N.D. |

### EXAMPLE 3: Effect of water content on gentamicin and vancomycin stability

The effect of residual water content on gentamicin and vancomycin stability of the formulations of EXAMPLE 1 was evaluated by a 60 min autoclave treatment. As summarized in Table 3 below, it was found that vancomycin had reduced stability in terms of recovery or purity at the higher residual water level. No significant effect of water on gentamicin stability was observed in the same range.

**Table 3:**

| Effect Of Water Content On Gentamicin And Vancomycin Stability | | | | |
|---|---|---|---|---|
| ID | | Vanco | | Genta |
| | | Recovery (% over Pre-autoclave) | Purity (%) | Recovery (% over Pre-autoclave) |
| EXAMPLE 1* (0.76% H₂O) | Pre-autoclave | 69.4 | 89.2 | 67.7 |
| | Autoclave | | 68.8 | |
| EXAMPLE 1* (1.26% H₂O) | Pre-autoclave | 65.7 | 89.9 | 80.2 |
| | Autoclave | | 64.5 | |
| Example 1* (1.76% H₂O) | Pre-autoclave | 62.0 | 89.5 | / |
| | Autoclave | | 63.2 | |
| Example 1* (2.26% H₂O) | Pre-autoclave | 60.5 | 88.8 | 77.5 |
| | Autoclave | | 58.8 | |
| | Autoclave | | 69.4 | |

| | | | | |
|---|---|---|---|---|
| * pH 5.7 | | | | |

### EXAMPLE 4: pH-stability and pH-solubility Profiles Of Gentamicin And Vancomycin In EXAMPLE 1 Formulations

The pH adjusted formulations of EXAMPLE 1 (without water added) were placed at 2-8 deg C for appearance examination. (See Table 4 below.)

**Table 4:**

| Effect of pH on Appearance of EXAMPLE 1 Formulations | | | |
|---|---|---|---|
| pH | Water (%) | Appearance | |
| | | Before filtration | Supernatant at 2-8 °C |
| 3.21 | 0.17 | Clear | Clear |
| 5.54 | 0.15 | Hazy | Clear for 5-7 days, then hazy |
| 5.63 | 0.13 | | |
| 6.02 | 0.04 | | |
| 6.01 | 0.11 | | |
| 6.99 | 0.11 | | |
| 7.67 | 0.09 | | |

A pH-stability profile was generated heating the sample with a 60 minute autoclave treatment. (See Table 5 below.)

**Table 5:**

| Effect of pH on Stability of EXAMPLE 1 formulations | | | | | |
|---|---|---|---|---|---|
| pH | Water (%) | Recovery (% over the pre-treatment) | | Vancomycin Purity (%) | |
| | | Vancomycin | Gentamicin | Pre-treatment | Post-treatment |
| 3.21 | 0.17 | 82.9 | 94.1 | 91.6 | 79.4 |
| 5.54 | 0.15 | 82.3 | 79.6 | 91.5 | 81.6 |
| 5.63 | 0.13 | 78.0 | 81.6 | 90.4 | 73.6 |
| 6.02 | 0.04 | 77.0 | 79.6 | 90.5 | 74.3 |
| 6.01 | 0.11 | 80.1 | 82.7 | 89.6 | 75.0 |
| 6.99 | 0.11 | 80.5 | 73.1 | 90.7 | 76.2 |
| 7.67 | 0.09 | 77.4 | 75.3 | 91.2 | 77.1 |
| 5.99 | 0.201 | 80.8 | 84.3 | 87.8 | 71.5 |

The results indicated that:
(1) pH affected EXAMPLE 1 formulations' appearance. The formulation was clear at pH 3.2;
(2) pH affected gentamicin's stability in the formulation. A low pH (e.g., from pH of 3 to 4) is preferred for gentamicin stability; and
(3) pH did not affect vancomycin stability significantly.

### EXAMPLE 5: pH Stability Profile Of Gentamicin in EXAMPLE 1 Formulation between pH of 3.0 to 5.5

Samples of EXAMPLE 1 formulations (without water added) at three different pH levels within 3.0 to 5.5 were prepared. In addition, the effect of L-histidine on the stability of the formulation of EXAMPLE 1 was also tested comparing the formulation containing L-histidine with those that do not contain L-histidine. The stability of gentamicin and vancomycin was evaluated in the same way as set forth in EXAMPLE 3. It was found that
(1) Stability of gentamicin in the formulation is pH-dependant (gentamicin preferred a low pH (e.g., from pH of 3 to 4));
(2) Stability of vancomycin in the formulation is less pH-sensitive in the pH range studied;
(3) L-histidine increased gentamicin stability in the pH range studied; and
(4) L-histidine decreased vancomycin stability in the pH range studied.

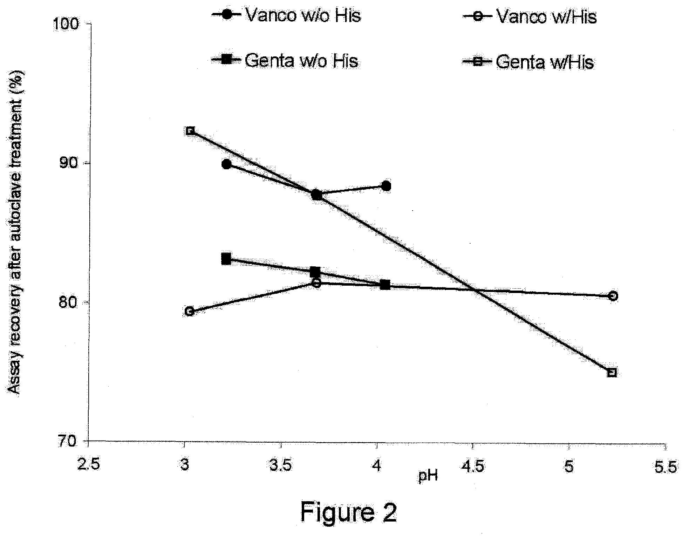

Figure 2 shows the assay recovery after the autoclave treatment.

### EXAMPLE 6: Another Formulation In Accordance With The Present Invention And The Process Of Making The Formulation

**Table 6:**

| List Of Ingredients Of Another Formulation | |
|---|---|
| In Accordance With The Present Invention | |
| Component | w/w% |
| Gentamicin sulfate | Equivalent to 1.675 % in the "USP Gentamicin Assay" value |
| Vancomycin hydrochloride | Equivalent to 1.876 % in the "USP Vancomycin Assay" value |
| Soy lecithin (PL90G) | 50.0 |
| Ethanol | 6.0 |
| Sesame oil | Qs to 100 |
| 1 N HCl pH | 3.3 |

A clear yellow sterile viscous depot (batch size: 1500 g), which contained less than 0.5 wt % of residual water having a pH of 3.3 was prepared by a multi-step process following the steps of: (1) emulsification, (2) microfluidization/homogenization, (3) lyophilization, (4) ethanol dilution, (5) pre-filtration, (6) ethanol removal and (7) filtration. Simple mixing of all of the ingredients listed above cannot form the formulation in accordance with this invention since it still not form a clear depot.

Detailed procedures for each of the above noted steps are as follows: First, water was added to gentamicin sulfate, vancomycin hydrochloride, to allow complete dissolution of gentamicin sulfate and vancomycin hydrochloride. Then, PHOSPHOLIPON® 90G (from Phospholipid GmbH) and sesame oil was added, followed by high shear mixing at 5000 rpm for 60 minutes to obtain a uniform emulsion. Then the pH of the emulsion was adjusted to 3.3 ± 0.2 by adding 1N of HCl. This was done by adding an appropriate amount of 1N HCl to the emulsion, followed by high shear mixing for 1 minute. Then, the measurement of pH was taken to ensure that the emulsion had a pH of 3.3 ± 0.2.

Subsequently, the emulsion was placed in a MICROFLUIDIZER to produce a monophasic solution. The average droplet size of the emulsion was measured using a laser light scattering device.

Then, the emulsion was lyophilized to remove water to obtain a dry paste with less than 0.5% residual water. Then the dry paste was mixed with dehydrated alcohol. The mixture was then sonicated in a 60-70 deg C water bath until a clear solution was obtained. Then the solution was cooled to room temperature, and was pre-filtered through a 0.65 micron sterile filter.

Then the alcohol from the solution was removed by blowing nitrogen gas until the residual amount of ethanol was 6.5 wt % - 7 wt % to obtain a viscous and clear gel. Dehydrated alcohol was added back as needed, if it was over dried.

In a biosafety hood, argon gas at 40 psi was applied to filter the viscous depot through a 0.2 micron filter to sterilize the formulation. Then, in a biosafety hood, filtered viscous depot was filled into a glass vial. EXAMPLE 7: In Vitro Release Profile

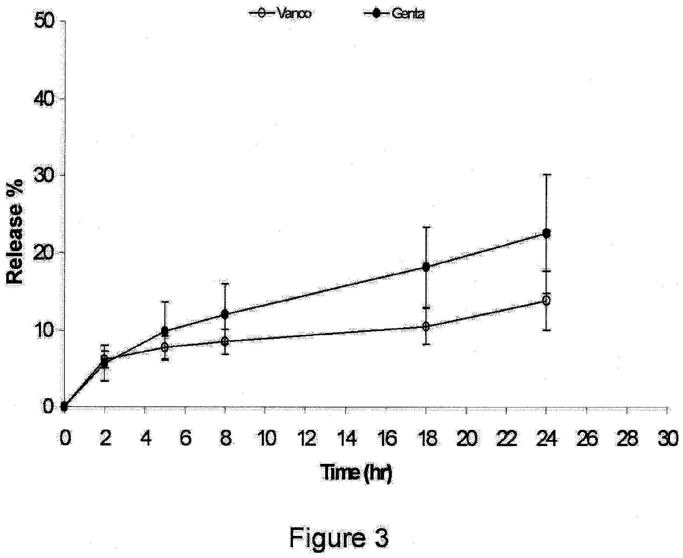

In vitro release profile of the formulation of EXAMPLE 6 containing gentamicin and vancomycin was measured using the USP method I using basket apparatus (100 rpm at 37 deg C). 1.36 g of EXAMPLE 6's formulation was filled in a 000 size capsule and the filled capsule was placed in 40 mesh basket with baffles. Figure 3 shows an in vitro release profile of gentamicin and vancomycin of the formulation of EXAMPLE 6 using USP method I.

### EXAMPLE 8: Pharmacokinetic Studies in Rabbits

New Zealand white rabbits were used to conduct pharmacokinetic ("PK") studies to evaluate the delivery of the formulations made in accordance with this invention. Two formulations were made in accordance with the procedures as set forth in EXAMPLE 1 and EXAMPLE 6, respectively, and administered into a surgical wound or subcutaneous pocket. Table 7 below shows the Rabbit PK study design in more detail:

**Table 7**

| Study | Gel Formulation | Vanco Dose (mg/kg) | Genta Dose (mg/kg) | Body Wt (kg) | Inj. Vol. (ml) | Vanco Con. (mg/g) | Genta Conc. (mg/g) |
|---|---|---|---|---|---|---|---|
| 1^{st} experiment | EXAMPLE 1 | 2.06 | 3.08 | 2.5 | 2.0 | 2.57 | 3.85 |
| 2^{nd} experiment | EXAMPLE 6 | 12.6 or 25.2 | 11.5 or 22.9 | 3.0 | 2 or 4 | 18.76 | 16.75 |

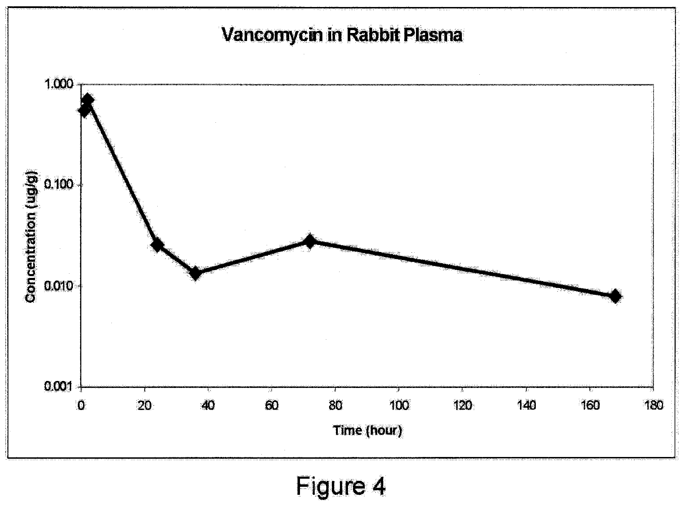

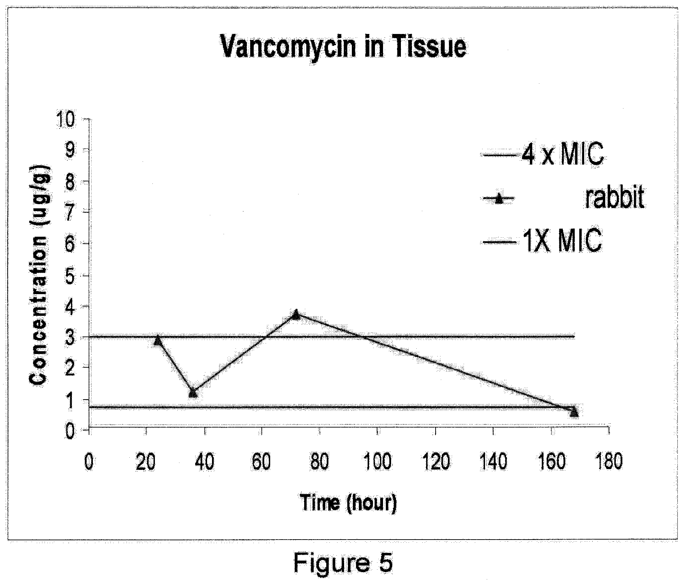

In a first experiment, two New Zealand white rabbits were tested. After wound instillation of the formulation of EXAMPLE 1, vancomycin and gentamicin were rapidly absorbed, with a plasma Tmax of 1-2 hours. Plasma Cmax concentrations were similar to those observed in the mouse. Plasma concentrations decreased to near the limit to quantitation by 36 hours. Tissue concentrations of vancomycin peaked at 72 hours and were above the Minimum Inhibitory Concentration (MIC) through 168 hours, as shown in Figs. 4 and 5.

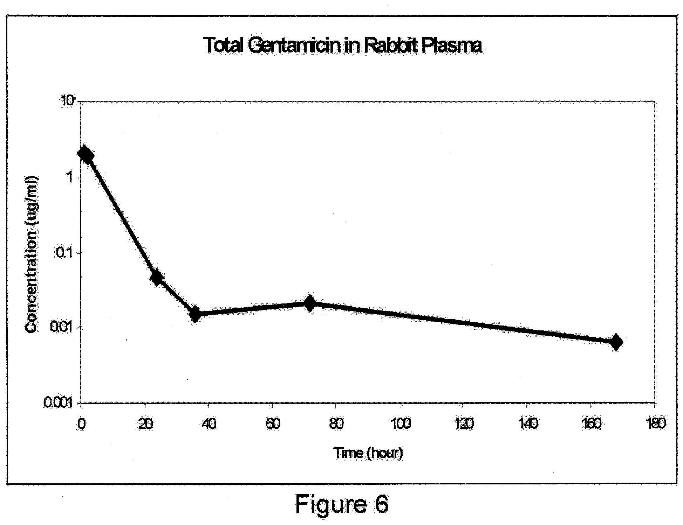

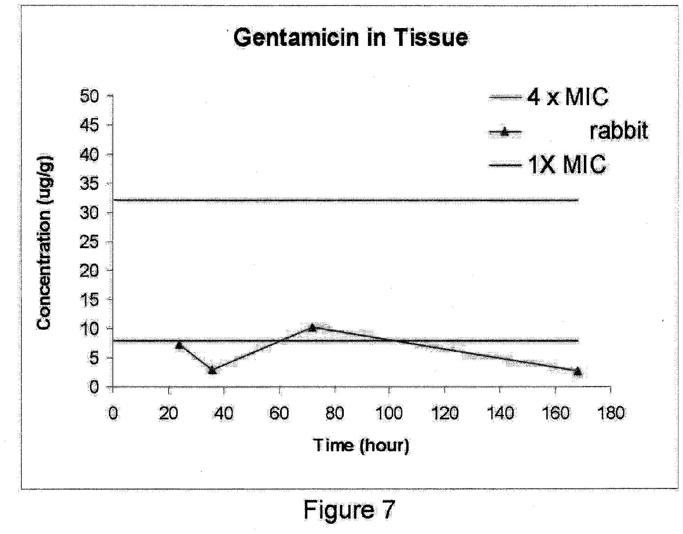

Tissue concentrations of gentamicin peaked at 72 hours and were at or below the MIC through 168 hours, as shown in Figs. 6 and 7.

Plasma and tissue analysis was performed by Liquid Chromatography/Mass spectrometry (LC-MS/MS) analysis and the Pharmacokinetic (PK) results of the formulation of EXAMPLE 1 are summarized in Tables 8 and 9, respectively below:

**Table 8:**

| Rabbit Plasma PK Parameters Of EXAMPLE 1 Formulation | | | | | | | |
|---|---|---|---|---|---|---|---|
| PK Parameters | Vanco | Genta C1a | Genta C1 | Genta C2/C2a | Genta Total | Vanco AUC/MIC | Genta Cₘₐₓ/MIC |
| Cₘₐₓ (µg/ml) | 0.702 | 0.278 | 1.063 | 0.746 | 2.085 | | 0.3 |
| Tₘₐₓ (hr) | 2 | 1 | 1 | 1 | 1 | | |
| AUC (hr* µg/ml) | 11.60 | 3.44 | 14.13 | 9.85 | 27.42 | 15.5 | |
| T_{1/2} (hr) | 39.16 | 10.50 | 23.24 | 23.83 | 22.91 | | |

**Table 9:**

| Rabbit Tissue PK Parameters Of EXAMPLE 1 Formulation | | | | | | | |
|---|---|---|---|---|---|---|---|
| PK Parameters | Vanco | Genta C1a | Genta C1 | Genta C2/C2a | Genta Total | Vanco AUC/MIC | Genta Cₘₐₓ/MIC |
| Cₘₐₓ (µg/ml) | 3.73 | 1.0525 | 5.865 | 3.23 | 10.1475 | | 1.3 |
| Tₘₐₓ (hr) | 72 | 72 | 72 | 72 | 72 | | |
| AUC (hr* µg/ml) | 354.8 8 | 104.28 | 573.10 | 324.86 | 1002.25 | 473.2 | |
| T_{1/2} (hr) | 35.32 | 50.32 | 49.68 | 51.60 | 50.35 | | |

In a second experiment, six New Zealand rabbits (Group I) were tested by wound instillation of the formulation of EXAMPLE 6 containing the dose of 12.6 mg/kg of vancomycin and 11.46 mg/kg of gentamicin; and six additional New Zealand rabbits (Group II) were tested by wound instillation of the formulation of EXAMPLE 6 containing the dose of 25.2 mg/kg of vancomycin and 22.9 mg/kg of gentamicin.

The lower concentration (Group I) gel averaged 4 µg/g for both vancomycin and gentamicin total in the wound site, while the higher concentration gel (Group II) averaged 26 and 19 4 µg/g for vancomycin and gentamicin, respectively, which are greater than four times MIC (minimum inhibitory concentration) values. Plasma concentrations of vancomycin and gentamicin from the MPI study of the formulation of EXAMPLE 6 exhibited vancomycin AUC/MIC (area under the concentration curve/minimum inhibitory concentration) ratios greater than 400 at both doses and gentamicin Cmax/MIC (maximum concentration/minimum inhibitory concentration) ratios greater than 800 at both doses.

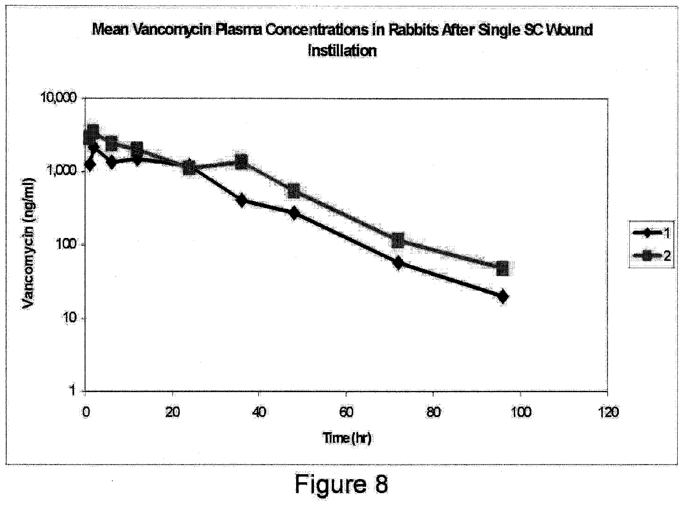

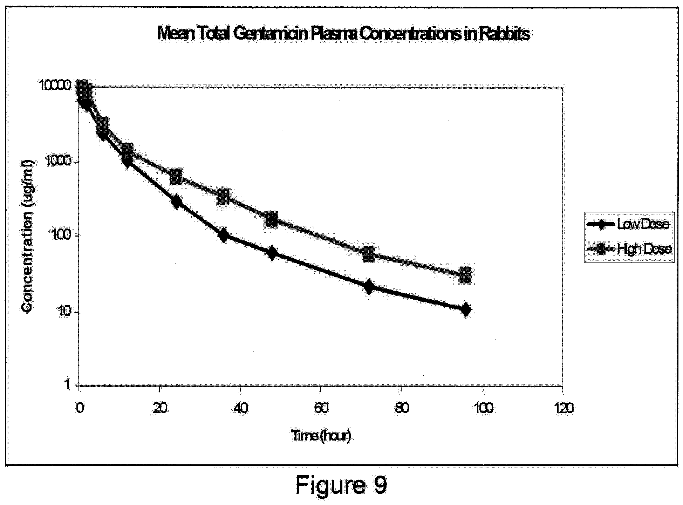

Fig. 8 illustrates mean vancomycin plasma concentrations in rabbits after single subcutaneous (SC) wound instillation and Fig. 9 illustrates mean total gentamicin plasma concentration in rabbits.

Plasma and tissue analysis was performed by LC-MS/MS analysis, and the PK results of the formulation of EXAMPLE 6 are summarized in Table 10 below:

**Table 10**

| | Vanco | | Genta C1 | | Genta C1a | | Genta C2+C2a | | Total Genta | | Vanco | | Total Genta | |
|---|---|---|---|---|---|---|---|---|---|---|---|---|---|---|
| Grp | 1 | 2 | 1 | 2 | 1 | 2 | 1 | 2 | 1 | 2 | AUC/MIC | | Cₘₐₓ/MIC | |
| | Mean | Mean | Mean | Mean | Mean | Mean | Mean | Mean | Mean | Mean | 1 | 2 | 1 | 2 |
| Cₘₐₓ | 3125 | 3150 | 2737 | 3586 | 769 | 1023 | 3188 | 4318 | 6679 | 8927 | | | 835 | 1116 |
| Tₘₐₓ | 3 | 2 | 1 | 1 | 1 | 1 | 1 | 1 | 1 | 1 | | | | |
| AUC last | 47392 | 60114 | 19216 | 25447 | 5463 | 7351 | 23286 | 31652 | 47966 | 64451 | 63190 | 80151 | | |

The key difference between the formulation of EXAMPLE 6 (high strength) and the formulation of EXAMPLE 1 (lower strength) is that although the PK profiles of these two formulations in small animals, such as mice, were similar, there was a greater difference in performance when tested on larger animals, such as rabbits, since the tissue concentrations for the formulation of EXAMPLE 1 fell below the 4 times MIC value sooner, therefore describing a lower area under the concentration curve (AUC) with respect to the time/area spent 4 times over the MIC.

### Comparative Example 1

| Ingredients | Weight % |
|---|---|
| Gentamicin sulfate | 3 |
| Vancomycin hydrochloride | 2 |
| Phospholipon 90G | 63 |
| Sesame oil | 27 |
| Ethanol | 5 |
| TOTAL | 100.00 |

Comparative Examples 1 was produced using the same methods as Examples 1 or 6, except that the homogenization, ethanol removal and/or pre-filtration steps were not performed.

Comparative Example 1 formed an opaque hard paste after lyophilization and was not suitable for injection even after adding ethanol.

### EXAMPLE 9: Wound Treatment Studies in Rats

Adult male Sprague-Dawley rats were used in this study. This study was conducted under a protocol in compliance with the Animal Welfare Act, the implementing Animal Welfare Regulations and in accordance with the principles of the Guide for the Care of Use of Laboratory. There were three study groups each containing ten animals. The first group received no treatment and the wound was closed after irrigation. The second group received four 3 mm PMMA beads containing approximately 28 mg of vancomycin and 32 mg of tobramycin which were placed into the wound after irrigation and before closure. The third group received 1 ml of formulation prepared in accordance with Example 1 containing approximately 17 mg of vancomycin and 19 mg of gentamicin placed into the wound after irrigation and before closure.

A contaminated open femoral defect was created using the following technique: Adult male Sprague-Dawley rats were anesthetized with isoflurane and prepared for surgery; their right femoral shafts were exposed and stabilized with a bespoke polyoxymethylene plate, secured with six threaded K-wires. A 6-mm defect was then created in the mid-shaft with a reticulating saw, cooled with saline. The defect was contaminated with 30 mg of sterile bovine collagen soaked with 1x10⁵ Colony Forming Units (CFUs) of *Staphylococcus aureus* in 0.5 ml of saline. The (Xenogen 36) strain of *S. aureus* used derived from ATCC 49525 originally from a septic human patient (Caliper LifeSciences, CA USA).

The wounds were closed in layers and the animals recovered. Six hours after the initial 'injury', the animals were re-anesthetized, their wounds opened, debrided with careful removal of all contamination and irrigated with 60 ml of sterile saline delivered at low pressure. Their wounds were again closed in layers. The animals recovered, and were allowed full mobility, food and water.

Fourteen days after injury the animals were euthanized. The femur and hardware were stripped of soft tissue and separated. The bone tissue was snap frozen in liquid nitrogen and crushed. Bone and implant samples were then sent separately for standard quantitative microbiological analysis. Briefly, crushed bone samples were homogenized with 10ml saline in an agitator, similarly implant specimens were rinsed with 10ml of saline in an agitator; then aliquots from individual specimens were sequentially diluted and spread onto Tryptic-Soy-Agar plates. After overnight incubation at 37°C, bacterial colonies were counted; the threshold of detectability was 30 CFU/g.

Antibiotic PMMA beads were manufactured under sterile conditions using Palacos R (Zimmer, Dover OH) arthroplasty cement. 40 g of MMA co-polymer powder was blended with 2.0 g of vancomycin and 2.4 g tobramycin (Sigma-Aldrich) then mixed with 20 ml of MMA monomer liquid. A 3 mm mold was then used to create beads weighing approximately 200 mg and containing 7 mg vancomycin and 8 mg tobramycin each. Four beads therefore represented a dose of 28 mg of vancomycin and 32 mg of tobramycin.

There were bacteria in all of the animals in the control group that received no antibiotics (study group 1) while only half of the animals treated with formulation prepared in accordance with Example 1 had detectable bacteria (study group 3). The formulation prepared in accordance with Example 1 (study group 3) was significantly superior to antibiotic beads (study group 2) with respect to reducing number of animals with bacteria detectible either in bone or on implants. Bacterial quantification demonstrated the superiority of formulation prepared in accordance with Example 1 (study group 3) compared to antibiotics beads (study group 2). The bacterial counts were very similar between the animals treated with antibiotic-beads (study group 2) and those that received no treatment (study group 1).

## Claims

1. A clear depot comprising a hydrophilic water-soluble pharmaceutically active agent(s) selected from the group consisting of a pharmaceutically acceptable salt of vancomycin, gentamicin, and a mixture thereof; water; a phospholipid; an oil; a pH adjusting agent; and a viscosity modifying agent selected from the group consisting of ethanol, isopropanol, and a mixture thereof; for use in the prevention and/or treatment of a wound infection; wherein the water present in the viscous depot is no more than 2 wt % relative to the total weight of the depot; and the pH of the depot is from 3 to 5.

2. The depot for use according to claim 1, wherein the wound is selected from the group consisting of chronic wound, acute wound, surgical wound, orthopedic wound, traumatic wound, combat wound and any combination thereof.

3. The depot for use according to claim 2, wherein the orthopedic wound is an injury to the musculoskeletal system.

4. The depot for use according to claim 2, wherein the traumatic wound is selected from an open wound to head, face, chest, abdomen, extremities , and/or external and/or injuries with torn and irregular edge with presence of foreign matter and/or non-viable tissue fragments, such as laceration, abrasions, puncture wounds, penetration wounds, and any combination thereof.

5. The depot for use according to claim 2, wherein the combat wound is selected from injuries inflicted by an explosive device and/or a weapon, gunshot wound, and any combination thereof.

6. The depot for use according to claim 1, which further comprise cleaning the wound to substantially remove a foreign material and/or non-viable tissue; and closing the wound.

7. The depot for use according to claim 6, wherein the step of closing the wound is performed using a suture, metal clips, staples or adhesive strips.

8. The depot for use according to any one of claim 1 to claim 7 which comprises administering the depot to a wound and using a vacuum-assisted wound closure system to the wound.

9. The depot for use according to any one of claim 1 to claim 8 which comprises cleaning the wound to substantially remove a foreign material and/or non-viable tissue; using a vacuum-assisted wound closure system to the wound; introducing the depot to a wound; and closing the wound.

10. The depot for use according to any one of claim 1 to claim 9 which comprises administering the depot either before, during or after closing of the wound using a suture, metal clip, staple, dressing, bandages and/or adhesive strips/tapes.

## Patentansprüche

1. Klares Depot, das Folgendes aufweist: (einen) hydrophile(n) wasserlösliche (n) pharmazeutische(n) Wirkstoff(e), der aus der Gruppe bestehend aus einem pharmazeutisch akzeptablen Salz von Vancomycin, Gentamicin und einem Gemisch davon ausgewählt ist; Wasser; ein Phospholipid; ein Öl; ein pH-Einstellmittel und ein Viskositätsmodifizierungsmittel, das aus der Gruppe bestehend aus Ethanol, Isopropanol und einem Gemisch davon ausgewählt ist; zur Verwendung bei der Verhütung und/oder Behandlung einer Wundinfektion; wobei das in dem viskosen Depot anwesende Wasser höchstens 2 Gew.-% bezüglich des Gesamtgewichts des Depots ist und der pH-Wert des Depots von 3 bis 5 ist.

2. Depot zur Verwendung nach Anspruch 1, wobei die Wunde aus der Gruppe bestehend aus chronischer Wunde, akuter Wunde, chirurgischer Wunde, orthopädischer Wunde, traumatischer Wunde, Verwundung und jedweder Kombination davon ausgewählt ist.

3. Depot zur Verwendung nach Anspruch 2, wobei die orthopädische Wunde eine Verletzung des Muskel-Skelett-Systems ist.

4. Depot zur Verwendung nach Anspruch 2, wobei die traumatische Wunde aus einer offenen Wunde an Kopf, Gesicht, Brust, Bauch, Gliedmaßen und/oder äußerlich und/oder Verletzungen mit eingerissenem und unregelmäßigem Wundrand mit Anwesenheit von Fremdmaterial und/oder nicht lebensfähigen Gewebefragmenten, wie etwa Risswunden, Schürfwunden, Stichwunden, Pfählungsverletzungen, und jedweder Kombination davon ausgewählt ist.

5. Depot zur Verwendung nach Anspruch 2, wobei die Verwundung aus durch einen Sprengkörper und/oder eine Waffe verursachten Verletzungen, Schusswunden und jedweder Kombination davon ausgewählt ist.

6. Depot zur Verwendung nach Anspruch 1, die ferner das Reinigen der Wunde, um Fremdkörper und/oder nicht lebensfähiges Gewebe im Wesentlichen zu entfernen, und das Schließen der Wunde aufweist.

7. Depot zur Verwendung nach Anspruch 6, wobei der Schritt des Schließens der Wunde unter Verwendung von Nahtgut, Metallklammern, Heftklammern oder Klebstreifen durchgeführt wird.

8. Depot zur Verwendung nach einem der Ansprüche 1 bis 7, die das Applizieren des Depots auf eine Wunde und die Verwendung eines vakuumunterstützten Wundverschlusssystems an der Wunde aufweist.

9. Depot zur Verwendung nach einem der Ansprüche 1 bis 8, die das Reinigen der Wunde, um Fremdkörper und/oder nicht lebensfähiges Gewebe im Wesentlichen zu entfernen; das Verwenden eines vakuumunterstützten Wundverschlusssystems an der Wunde; das Einbringen des Depots in eine Wunde und das Schließen der Wunde aufweist.

10. Depot zur Verwendung nach einem der Ansprüche 1 bis 9, die das Applizieren des Depots entweder vor, während oder nach dem Schließen der Wunde unter Verwendung einer Naht, einer Metallklammer, einer Heftklammer, eines Verbands, von Binden und/oder Klebstreifen/-bändern aufweist.

## Revendications

1. Dépôt clair comprenant un ou des agents hydrophiles pharmaceutiquement actifs solubles dans l'eau sélectionné dans le groupe consistant en un sel pharmaceutiquement acceptable de vancomycine, gentamicine, et un mélange de ceux-ci ; un phospholipide ; une huile ; un agent d'ajustement du PH ; et un agent de modification de viscosité sélectionné dans le groupe consistant en éthanol, isopropanol, et un mélange de ceux-ci ; destiné à être utilisé dans la prévention et/ou le traitement d'une infection de plaie ; dans lequel l'eau présente dans le dépôt visqueux ne représente pas plus de 2 % en poids du poids total du dépôt ; et le pH du dépôt est compris entre 3 et 5.

2. Dépôt destiné à être utilisé selon la revendication 1, dans lequel la plaie est sélectionnée dans le groupe consistant en plaie chronique, plaie aiguë, plaie chirurgicale, plaie orthopédique, plaie traumatique, plaie de guerre et n'importe quelle combinaison de celles-ci.

3. Dépôt destiné à être utilisé selon la revendication 2, dans lequel la plaie orthopédique est une blessure du système musculo-squelettique.

4. Dépôt destiné à être utilisé selon la revendication 2, dans lequel la plaie traumatique est sélectionnée parmi une plaie ouverte à la tête, au visage, au torse, à l'abdomen, aux extrémités, et/ou externe et/ou des blessures à bord déchiré et irrégulier avec présence de corps étrangers et/ou fragments de tissus non viables, telles que lacérations, abrasions, plaies punctiformes, plaies par incision, et n'importe quelle combinaison de celles-ci.

5. Dépôt destiné à être utilisé selon la revendication 2, dans lequel la plaie de guerre est sélectionnée parmi les blessures infligées par un dispositif explosif et/ou une arme, une plaie par balle, et n'importe quelle combinaison de celles-ci.

6. Dépôt destiné à être utilisé selon la revendication 1, lequel comprend en outre le nettoyage de la plaie pour sensiblement éliminer un corps étranger et/ou un tissu non viable ; et la fermeture de la plaie.

7. Dépôt destiné à être utilisé selon la revendication 6, dans lequel l'étape de fermeture de la plaie est exécutée au moyen d'une suture, de clips métalliques, d'agrafes ou de bandes adhésives.

8. Dépôt destiné à être utilisé selon l'une quelconque de la revendication 1 à la revendication 7, lequel comprend l'administration du dépôt sur une plaie et l'utilisation d'un système de fermeture de plaie par pression négative sur la plaie.

9. Dépôt destiné à être utilisé selon l'une quelconque de la revendication 1 à la revendication 8 qui comprend le nettoyage de la plaie pour sensiblement éliminer un corps étranger et/ou un tissu non viable ; l'utilisation d'un système de fermeture de plaie par pression négative sur la plaie ; l'introduction du dépôt sur une plaie ; et la fermeture de la plaie.

10. Dépôt destiné à être utilisé selon l'une quelconque de la revendication 1 à la revendication 9 lequel comprend l'administration du dépôt avant, durant ou après la fermeture de la plaie au moyen d'une suture, d'un clip métallique, d'une agrafe, d'un pansement, de bandages et/ou de bandes/rubans adhésifs.
